# EUROPEAN PATENT SPECIFICATION

(11) **EP 1 073 448 B1**
(45) Date of publication and mention of the grant of the patent: **02.01.2002**
(21) Application number: 99919238.8
(22) Date of filing: 15.04.1999
(51) Int. Cl.: A61K 38/00, C12N 5/06, C12N 5/08

(54) **FERTILITY IMPROVING COMPOSITION AND APPLICATION THEREOF**
ZUSAMMENSETZUNG ZUR VERBESSERUNG DER FRUCHTBARKEIT UND IHRE VERWENDUNG
COMPOSITION FAVORISANT LA FECONDITE ET APPLICATION

(30) Priority: 27.04.1998 EP 98107602
(43) Date of publication of application: 07.02.2001
(73) Proprietor: NIKA HEALTH PRODUCTS LIMITED, FL-9490 Vaduz (LI)
(72) Inventor: KICZKA, Witold, Princeton, NJ 08540 (US)
(74) Representative: Büchel, Kurt F., Dr.
(86) International application number: EP9902539
(87) International publication number: WO9955354

(56) References cited:
- WO-A-96/21463
- DATABASE MEDLINE [Online] US NATIONAL LIBRARY OF MEDICINE (NLM), BETHESDA, MD, US KUZ'MIN M D ET AL: "[The diagnosis of male infertility based on the lysozyme level in sperm]. Diagnostika muzhskogo besplodiia po urovniu lizotsima v sperme." XP002078899 & LABORATORNOE DELO, (1991) (7) 39-41,

## Description

The present invention relates to applications of a dimerized form of lysozyme for the improvement of fertility of animal or human germ cells, e.g. oocytes and spermatocytes. It further relates to the prophylactic intervention or therapeutic treatment of fertility or reproductive disorders in animals or humans. The invention also relates to the use of a lysozyme dimer for the manufacture of a pharmaceutical composition suitable for such applications and to an improved method for the preservation of human or animal cells, and for a prolongation of the survival time of cells.

### BACKGROUND

The limitation of the practical use of lysozyme and other therapeutically active enzymes was overcome in the late eighties, when it was discovered that isolated dimerized forms of enzymes, while retaining all beneficial properties of known monomeric forms, exhibit no negative side effects when used in therapeutic doses. The antiviral and antibacterial compositions comprising as the active ingredient lysozyme dimer or other dimerized enzymes have been described in WO 89/11294, whose entire contents shall herewith be incorporated by reference.
Later on, further attractive features of lysozyme dimer were found and additional therapeutical applications of the drug were developed, especially for the treatment of bacterial and viral infections as disclosed in WO 94/01127, whose entire contents shall herewith be incorporated by reference. The inventor observed certain immunomodulating effects of the dimerized lysozyme, particularly concerning the modulation of cytokine levels, confirmed by *in vitro* and *in vivo* experiments. The lysozyme dimer is able - amongst others - to modulate the synthesis of TNFα, IL-2, IL-6 and INFα, and to activate phagocytosis and the immunological mechanisms connected therewith. The lysozyme dimer is particularly useful for the treatment and prophylaxis of diseases associated with excessively high levels of TNFα.

It could also be successfully demonstrated that lysozyme dimer compositions display remarkable potency in the inhibition or even total prevention of leukemic cell proliferation *in vivo,* particularly in the case of virus induced lymphatic leukemia. Further investigations led to the manufacture of pharmaceutical compositions comprising lysozyme dimer as the active component applicable in cases of hair growth disorders, particularly hair growth disorders based on immunological malfunctions or dysfunctions, or in preventing or treating diseases connected with a suppressed immune system. These remarkable effects of the lysozyme dimer have been disclosed in WO 96/21463, whose entire contents shall herewith be incorporated by reference.

### SUMMARY OF THE INVENTION

Surprisingly, further investigations revealed that the lysozyme dimer was also capable of positively affecting the preservation, particularly the freeze-preservation of animal semen. It is of considerable importance - also in terms of commercial benefit - to have animal and human sperm samples properly preserved in order to maintain motility and fertility of the spermatozoa ( = sperm cells) and to keep the number of morphologically changed (i.e. pathologically altered) sperm cells as low as possible.

It is well known in the art to use various additives and adjuvants in admixture with the semen to facilitate freeze-preservation and protect the cells during thawing. For instance, the Russian patent application SU-604556-A teaches the preparation of a synthetic aqueous medium for diluting and freezing agricultural animal sperm which comprises gum arabic, glycerol, lactose and 10 to 20 % by weight of chicken egg yolk.

The adjuvant effect of the lysozyme dimer and its contribution to an improvement in the preservation at various temperatures - though most frequently involving at least one deep-freezing step - of animal or human cells including tissue cells, blood cells, and sperm cells is proven by analytical assessment of the most relevant parameters of biological activity or viability of the preserved and stored biological material, particularly after thawing of deep-frozen samples. In case of animal or human sperm cells the improvements in the percentage of spermatozoa with normal motility, spermatozoon morphology, biochemical and biological activity are determined after thawing.

The treatment of animal or human cell suspensions with the lysozyme dimer prior to storage and storing the cells together with the lysozyme dimer is particularly preferred, regardless of the temperature of storage, e.g., above or below freezing point temperature. Beside the adjuvant effect of the lysozyme dimer - the obtained results indicate that it can adhere to and stabilize the cell membranes of spermatozoa and other eukaryotic cells even at very low temperatures - it is probably also its antibiotic activity and its inhibition of possible virus penetration that adds in the overall preservation improving efficacy, particularly during liquid storage. When storing at temperatures below the freezing point it is preferred to add the lysozyme dimer to the cell suspensions prior to the freezing step.

The present invention is also directed to the use of a lysozyme dimer for the manufacture of a pharmaceutical composition for the improvement of fertility of oocytes, more particularly to the improved maturation of oocytes and the improved subsequent development of the fertilized egg cell and early embryo. In vitro experiments on bovine germ cells revealed that the presence of lysozyme dimer in the maturation medium in which the fertilization of the bovine oocytes took place had a beneficial effect on the fertilization rate and the quality of the resulting embryos. It was also found that the addition of a portion of the supernatant of a cell culture of polymorphonuclear cells (PMN), or of lymphocytes, grown in the presence of a combination of lysozyme dimer and a mitogen such as LPS, PHA, or ConA, to the maturation medium of the oocytes had a similar advantageous effect on the fertilization rate and the embryo development.

Another objective of the present invention is the use of a lysozyme dimer for the manufacture of a pharmaceutical composition for the improvement of animal or human sperm quality *in vivo* as well as the prophylactic intervention or therapeutic treatment of fertility or reproduction disorders of any known or unknown etiology, particularly of male fertility disorders having an immunological, hormonal or metabolic origin. In the most preferred embodiment the present invention is directed to the use of a lysozyme dimer for the manufacture of a pharmaceutical composition for the prophylaxis or therapeutic treatment of oligospermia in animals or humans by administering an effective dose of lysozyme dimer to an animal or human individual at risk of or subject to such a fertility disorder to avoid or relieve such a condition.

Oligospermia is characterized by a pathologically decreased concentration of spermatozoa and an abnormally high percentage of morphologically changed spermatozoa. It may be caused - amongst others - by hormonal, immunological or metabolic dysfunctions affecting the spermiogenesis, i.e. the production and/or riping of the spermatozoa. It was successfully demonstrated *in vivo* that after intramuscular injection of a pharmaceutical composition comprising lysozyme dimer as the active component to various male domestic animals the sperm quality was considerably improved comprising but not limited to a drastic increase of sperm cell concentration and a remarkable decrease in the quantity of morphologically changed spermatozoa. It is believed that the wide range of immunomodulating actions of lysozyme dimer significantly contribute to the observed beneficial effects.

The terms "modulating" and "modulation" used herein with regard to the lysozyme dimer's action on the animal or human immune system shall express the bifunctional, immune system balancing nature of that action. In the case of a suppressed immunological status of an individual, e.g. caused by infectious diseases, chemotherapy, stress, pollutants and the like, the lysozyme dimer strengthens the immune system and usually restores a physiologically normal level of immunological defense capacity or at least significantly contributes to such restoration through actions comprising *inter alia* augmentation of blood or serum levels of certain cytokines including interferon and some interleukins, and/or enhancement of phagocytic activity of the various phagocytizing kinds of cells of the immune system such as, for instance, monocytes, polymorphonuclear cells (PMN), and macrophages. On the other hand, if the immune system is overly challenged by toxic or otherwise damaging agents or itself produces a pathologically exaggerated immune response, the administration of lysozyme dimer may calm down the overwhelming immune response through actions comprising, for instance, decreasing dangerously high tumor necrosis factor (TNF) levels, decreasing high fever to normal body temperature, and/or decreasing blood or serum levels of the various well known forms of free oxidative radicals occurring in biological systems (including oxygen, hydroxyl, and nitrogen oxide radicals). It is a further object of the present invention to provide for a method to treat female individuals that suffer from a disturbed fertility, which may be caused, for instance, by a postpartum inflammation of the uterus endometrium, as is frequently the case with domestic animals, in particular with cows. It could be demonstrated in *in vivo* studies that intrauterine infusion of a lysozyme dimer composition decreased the activity of endogenic prostaglandin. Also, a faster maturation of ovarian follicles, faster luteinization and a faster decrease of the P-4 progesterone level at a simultaneous increase in estradiol level was observed in the lysozyme dimer treated cows compared to the untreated control group. These dynamic changes together with a rapid breakdown of the prostaglandin F₂ production had in effect that the lysozyme dimer treated cows were able to resume the estrous cycle about 11 ± 3 days earlier than cows from the control group.

The present invention is further directed to the use of lysozyme dimer for the manufacture of a pharmaceutical composition to be applied for the aforementioned purposes. It is preferred that the lysozyme dimer be administrable to the human or animal individual in a single or repeated dose of about 0.005 to 5 mg/kg, preferably of about 0.01 to 1 mg/kg body weight, corresponding to about 0.07 to 70 or 0.14 to 14 µg lysozyme dimer per ml blood of a human individual, respectively (calculated on the basis of a 70 kg man having 5 liters blood). Advantageously, the drug is administrable in the form of an injection solution containing the lysozyme dimer at a concentration of 0.01 to 10 mg/ml, preferably 0.1 to 0.5 mg/ml of the solution.

It is further preferred that the applied lysozyme dimer is a dimerization product obtained by protein crosslinking of lysozyme monomers, which contains about 10 % by weight or less of undesired byproducts, particularly of monomeric and oligomeric forms of lysozyme. The lysozyme monomers are preferably derived from hen eggs, but may also be obtained from other natural sources comprising humans, animals, plants and microorganisms or may be manufactured by chemical synthesis or by genetic engineering methods.

The term Lydium-KLP as used herein refers to a liquid composition, preferably an injection solution, comprising a suitable solvent, a suitable preservative, and as the active drug lysozyme dimer - obtained via chemical crosslinking of egg white lysozyme - at a concentration of 0.01 to 10 mg/ml, preferably 0.1 to 0.5 mg/ml of the solution.

### BRIEF DESCRIPTION OF THE DRAWINGS

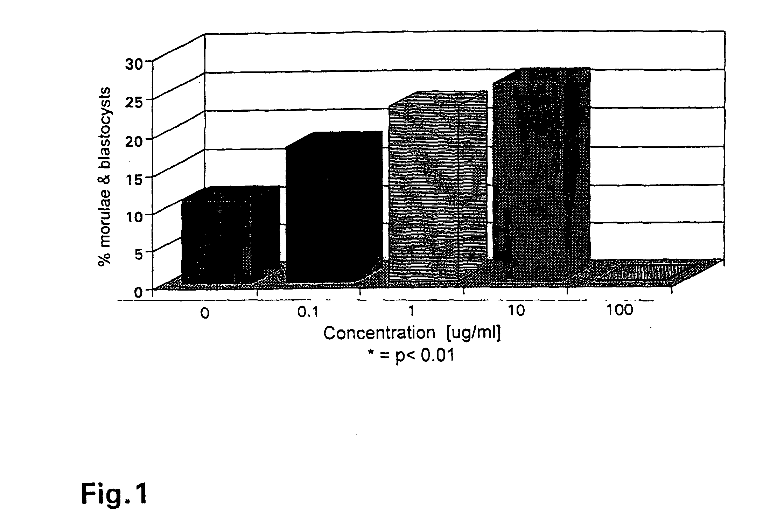

- Fig. 1: is a graph showing the development of oocytes fertilized in the presence of different concentrations of lysozyme dimer (as described in Example 6 hereinafter).

In order that the invention described herein may be more fully understood, the following examples are set forth. The examples are for illustrative purposes only and are not to be construed as limiting this invention in any respect.

### Example 1: Effect of lysozyme dimer on the preservation of semen

Investigations were performed on the effect of lysozyme dimer on boar semen preserved at low temperatures. The experiments were conducted under clinical conditions on six boars of different breeds: one "Duroc", three "Hampshire", one "Pietrain" and one "WBP". The animals were used for artificial insemination once a week.

Fresh semen was treated with lysozyme dimer immediately after collection in the doses of 1, 2 and 4 µg per 1 ml of semen. The ejaculate was thoroughly tested at all phases of the experiment, i. e. before and after adding lysozyme dimer to the semen, before freezing the semen, and after thawing. The following factors were assayed; spermatozoon concentration, percentage of spermatozoa with normal motility, spermatozoon morphology (including acrosomes), and survival rates in various diluents and at various temperatures. Biochemical assays included: GOT (glutamate-oxaloacetate-transaminase), acrosin, hyaluronidase, acid phosphatase, and alkaline phosphatase.
The following methods of preservation by freezing were used: a) the ball method, b) freezing in 5 ml plastic round tubes, and freezing in 7 ml flat tubes. Preliminary results are presented in Tables 1, 2 and 3.

Table 1 shows that, unlike in the control group, when 4 µg of lysozyme dimer was added to 1ml of fresh semen, the percentage of spermatozoa with normal motility increased on average by 10 % after equilibration at 5° C and on average by 15 % after thawing of the semen.

Table 2 shows the effect of lysozyme dimer (4 µg per 1 ml of semen) on the percentage of spermatozoa with acrosomes that were damaged during the process of preservation. In the experimental sample the percentage of damaged acrosomes in spermatozoa is significantly lower than in the control samples (no lysozyme dimer added).

Table 3 shows the results of GOT activity assays at individual phases of the experiment. It was noted that lysozyme dimer reduced the discharge of the enzyme from the spermatozoa after thawing (429 U per 10⁹ spermatozoa on average) thus indicating less frequent occurrences of spermatozoon cell damages.

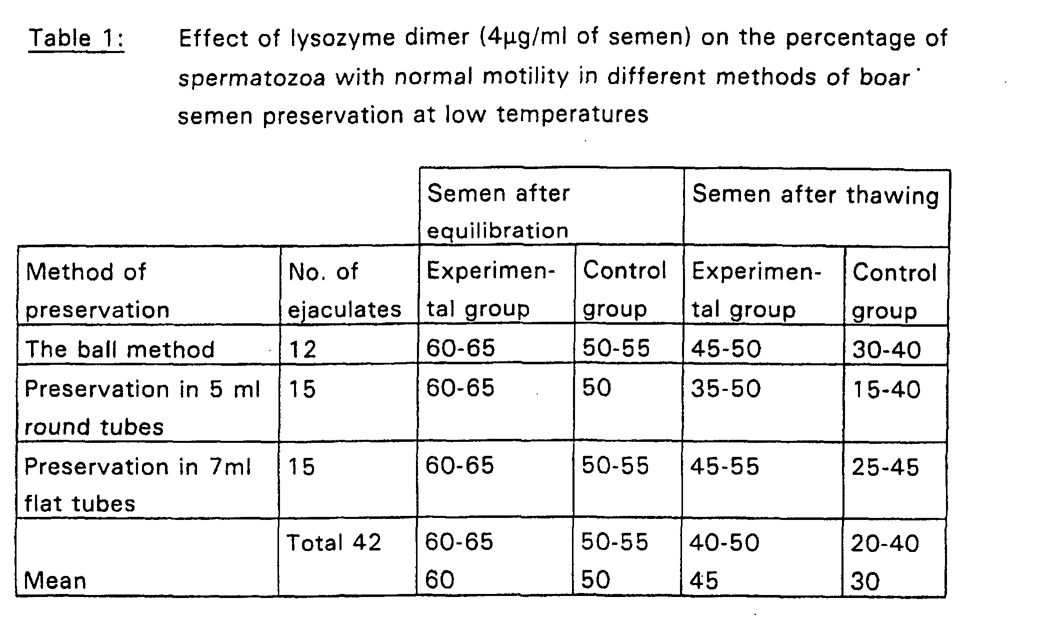

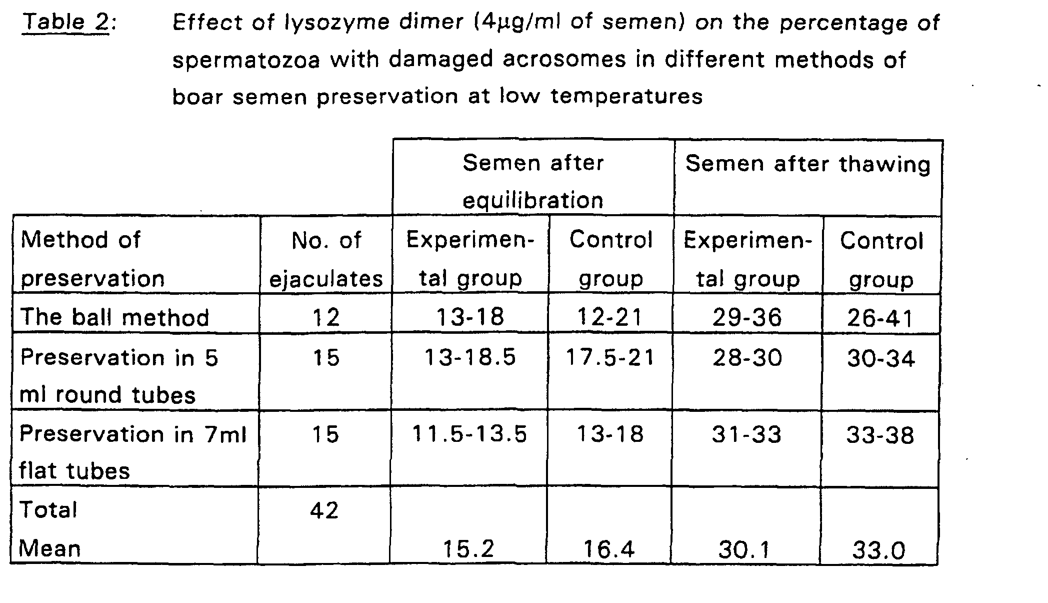

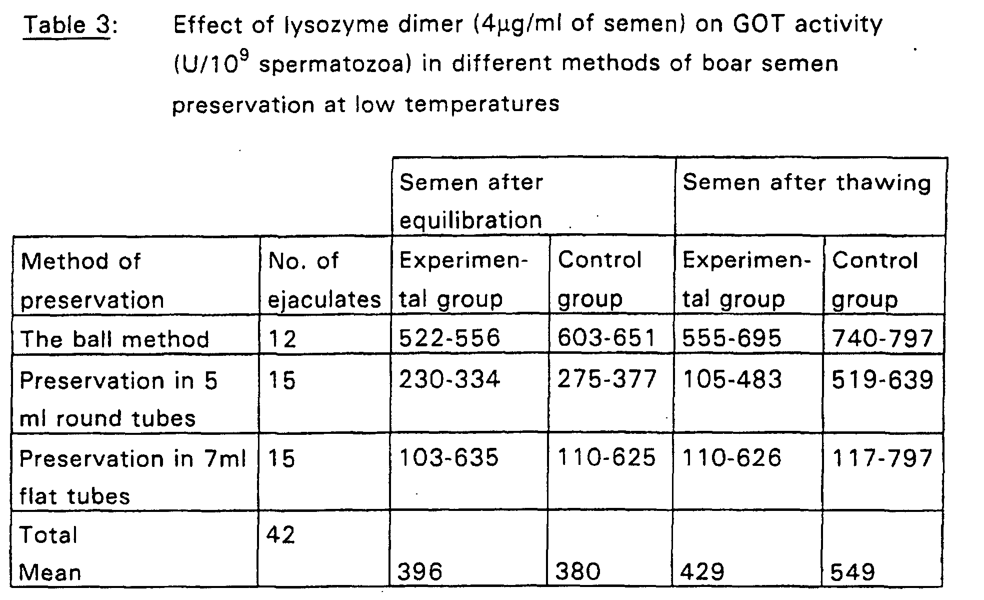

The promising results of the investigations described above indicate that lysozyme dimer can successfully be used in the preservation of animal or human sperm.

### Example 2: Effect of lysozyme dimer (KLP-602) on preservation of animal spermatozoa

Studies were carried out on semen of different males of domestic animals (6 boars, 5 dogs, 5 rams and 4 bucks). The lysozyme dimer preparation was applied to the fresh semen directly after collection and before conservation, in the following doses: 1, 2, 4, 5, 10, 15 and 20 µg/ml of semen. In all stages of the experiment (before and after adding lysozyme dimer to the semen, just before freezing and after thawing of the semen) a detailed examination of the semen was performed including determination of spermatozoa concentration, percentage of properly motile spermatozoa, morphology of the spermatozoa (including the acrosomes), survival rates at various dilutions and temperatures. The biochemical investigations included determination of GOT, hyaluronidase, and the activity of acidic and alkaline phosphatase. Freezing of the semen was performed using various methods of cryoprotection, such as freezing in pellets, straws (0.25 ml), 5 ml round and 1.7 ml flat tubes. The addition of 4 - 10 µg lysozyme dimer to 1 ml of the fresh semen caused a real increase of spermatozoa with progressive motility after thawing, a decrease of the number of damaged acrosomes and reduced release of GOT from preserved spermatozoa for all animals. The results obtained indicate that addition of lysozyme dimer to fresh semen of different male animals can stabilize the cell membranes of spermatozoa for preservation at low temperatures.

### Example 3: Efficacy of lysozyme dimer in the treatment of oligospermia in boars

The experiment was performed on a six-year old boar with symptoms of oligospermia that persisted for 1.5 months, and with a high percentage of morphologically changed spermatozoa caused by atrophy of the right testis. The boar was administered intramuscularly 10 ml of Lydium-KLP comprising 2 mg of lysozyme dimer as the active ingredient, corresponding to a dosage of approximately 0.020 mg/kg body weight of the animal.

Before treatment, the spermatozoa concentration per ejaculate volume unit gradually decreased from 145.000 to 65.000 per mm³ and morphological changes in spermatozoa varied from 25 % to 50 % (mean value 40 %). 51 days after injection of Lydium-KLP, the spermatozoon concentration increased from 100.000 to 150.000 per mm³ and the morphological changes in spermatozoa were within the range of 18 to 21 % (mean value 19,5 %).
It can thus be concluded therefrom that Lydium-KLP seems to be a remarkable agent in the treatment of oligospermia in animals or humans.

### Example 4: Effect of lysozyme dimer on the survival of sperm cells

This example describes the effect of lysozyme dimer on the survival and biological activity of spermatozoa of boars and bulls. The study comprises determination of the:
- influence of addition of lysozyme dimer to fresh semen of boars and bulls without subsequent conservation;
- influence of lysozyme dimer on the process of cryoconservation of semen of these males, and
- influence of the administration of a pharmaceutical lysozyme dimer composition (Lydium-KLP) on the fertilizing capacity of bulls and boars with poor quality of semen.
The studies were carried out on 75 boars and 65 bulls and their ejaculates.

### a) In vitro study:

In the course of the study, a most effective dose of lysozyme dimer added to the fresh semen was found to be 5 µg per 1 ml of semen. Lower doses, e.g. 1 and 3 µg/ml of semen had reduced or no biological activity. Similarly, doses higher than 5 µg/ml of semen did not produce univocal, repeatable biological effects. Therefore, in this study the addition of 5 µg of lysozyme dimer per 1 ml of fresh semen was used.

The addition of lysozyme dimer to fresh semen (without subjecting the semen to subsequent freezing) caused an average elongation of the survival time of spermatozoa of boars of about 6.8 ± 1.5 hours, and of the bulls of about 3 ± 1.2 h, in comparison to control samples.

Similarly, the addition of 5 µg of lysozyme dimer to semen with subsequent cryoconservation and storage for 30 days in liquid nitrogen (-196°C) and subsequent unfreezing, had an advantageous influence on the elongation of the survival time of the sperm cells, which in examined ejaculates fluctuated from 6 to 14 h in comparison with analogous control samples.

Additionally, it was observed that in the lysozyme dimer treated ejaculates, particularly in the semen of boars, the percentage of spermatozoa with normal motility was about 10 - 15% higher than in the untreated controls. Also, the number of spermatozoa with primary defects was about 20 - 25% lower in comparison to analogous trials without the addition of lysozyme dimer.

Moreover, in the lysozyme dimer treated group the observed lower concentration of spermatozoa with damages of acrosomes was statistically significant (p < 0,01). This effect was particularly visible with the semen of boars: in control samples the percentage of spermatozoa with damages of acrosome fluctuated between 35 and 60%, whereas in the experimental samples the percentage did not exceed 28%.

Further, the addition of lysozyme dimer to semen with subsequent cryoconservation and thawing caused a statistically significant (p < 0.05) decrease of sperm cell destruction, as measured by means of discharged transaminases (AspAT). In the boar semen, a reduction in damaged cells of about 65 ± 8%, in semen of bulls of about 48 ± 11%, in comparison to control samples, was observed.

### In vivo study:

20 boars and 15 bulls having a poor quality of semen (low concentration of spermatozoa in the ejaculate, high percentage of cells with primary defects and damaged acrosomes, low percentage of spermatozoa with normal motility) and as a consequence thereof a considerably reduced reproductive capacity were subjected to lysozyme dimer treatment:
A pharmaceutical lysozyme dimer composition (Lydium-KLP) was administered intramuscularly to the animals at a dose of 0.02 mg lysozyme dimer per kg body weight. The preparation was applied thrice in intervals of 10 days. The ejaculate was examined once a week in the period from first administration until the 95th day after the last administration. It was found that positive changes in the microscopic appearance of the boar semen developed - on average - after 40 ± 5 days after the first application of Lydium-KLP, and of bull semen after 50 ± 4 days and was maintained up to 48 ± 6 days in the boar and up to 65 ± 7 days in bulls. These changes were manifested by an increase of the spermatozoa concentration of about 25 - 40% in comparison to the initial concentration at time zero, an increase of the percentage of sperm cells with normal motility of about 10 to 26% compared to the situation at time zero.

Distinct changes were also observed with regard to the number of spermatozoa with primary defects. In the experimental group, their number decreased by about 20 to 30% in comparison to the control groups.

### c) fertility testing:

In the experimental period, a biological evaluation of the boar and bull semen either patronised by lysozyme dimer or obtained from males that received Lydium-KLP treatment was undertaken in local tests. Studies were carried out on 80 cows and 100 sows. It was shown, that the non-repetition factor (NR) in cows inseminated by semen protected by the addition of lysozyme dimer was 87.5 ± 8%. In the population of control cows this value was 70 ± 9%. In the population of sows inseminated by lysozyme dimer protected semen the value was 86 ± 5% and in the sow population of the control group it was 74 ± 6%.

Particularly positive biological effects were obtained after insemination of sows using semen of boars that had received a Lydium-KLP treatment as described above. Prior to this immunostimulation, the efficacy of sow insemination by ejaculates of these boars did never exceed 35 - 40%; after immunostimulation of the boars by the aforementioned lysozyme dimer therapy, the insemination efficacy increased to 78 - 85% during the experimental period.

### Example 5: Use of lysozyme dimer in the treatment of impaired fertility in animals

This example describes an experimental trial to demonstrate and explain the activity and involved modes of action of lysozyme dimer towards endometritis in cows.
The studies were carried out on 120 cows with postpartum inflammation of the uterus endometrium (endometritis puerparalis). The experimental group comprised 60 cows randomly selected. The control group comprised the same number of cows. Cows from the experimental group received 2 mg of lysozyme dimer in 40 - 50 ml of a 5% glucose solution intrauterinely using a catheter. Cows from the control group received an infusion of antibiotics (Amoksiklav). The gynecological examinations took into consideration: state of uterus and uterine cervix, course of involution and term of finishing, filling of the uterine cavity - amount, character and smell of discharges, estimation of the ovary activity. Laboratory investigations carried out on 20 cows from every group (Σ = 40 females) included: determination of factors of the cellular immunity, using the blastic transformation test under the influence of LF-7, phagocytosis test according to Wright, reduction of nitrotetrazolium blue (NBT), esterase blue, determination of immunity factors of the humoral immunity - total protein and its fractions, leukocyte pattern, determination of the hormone level of the ovarian cycle - progesterone (P-4) and estradiol - and activity of the metabolite of PGF2 alpha (PGFM). The efficacy of the applied therapeutical procedure was estimated on the basis of the obtained values from the investigated immunological factors, the dynamics of changes of PGF2 alpha, the level of progesterone of the ovarian cycle, and the calculated fertility indexes such as length of the period from calving to first heat, pregnancy index, insemination index and length of the intergestation period.

The laboratory investigations showed a beneficial immunomodulating influence of intravenously administered Lydium-KLP on the values of the analyzed factors of the cellular immunity. In cows of the experimental group an increase of the phagocytosis index of about 15 to 21 %, an increase of the phagocytic activity and transformation ability of about 10 to 18% on average, and a distinct increase of the gammaglobulin fraction between the 3rd and 5th day after infusion of Lydium-KLP was observed.
In the control group of cows receiving the antibiotics changes in the immunological activity were detected and the values of the investigated factors indicated the appearance of a postpartum immunological niche, observed also in cows in other studies.

The intrauterine infusion of Lydium-KLP decreased the activity of the endogenic prostaglandin, determined as PGFM. Particularly, distinct changes of the dynamics of this parameter are seen between 2 and 5 hours after lysozyme dimer application. Its activity decreases by about 70 - 85% in comparison to the value, that was observed before infusion of Lydium-KLP.

In cows receiving Lydium-KLP intravenously, an earlier maturation of ovarian follicles, an earlier luteinization, and a faster decrease in the P-4 level in comparison to analogous changes in the cows of the control group was observed. Simultaneously with the changes in the level of P-4, changes in the dynamics of the estradiols in blood were observed. Their increase correlated with the dynamics of disappearance of P-4. These changes and their faster rates in the cows from the experimental group had in effect that cows from this group came into the estrous cycle at about 11 ± 3 days earlier than cows from the control group.
The observed changes in the dynamics of the prostaglandin F2 production and particularly its fast breakdown seem to be the main outcome of the beneficial activity of Lydium-KLP in the therapy of endometritis in cows. The dynamics of changes in the progesterone and estradiol levels in blood will confirm these remarks.

### Example 6: Effect of lysozyme dimer (KLP-602) on bovine egg cells (oocytes) maturation and fertilization in vitro

The following experiments were performed:
A. Determination of the effect of different lysozyme dimer concentrations on maturation and in vitro fertilization of bovine egg cells (oocytes).
B. Evaluation of the lysozyme dimer influence on the interrelationship between either polymorphonuclear cells (PMNs) or lymphocytes, and oocyte maturation and early embryonic development.
C. Determination of the influence of different concentrations of lysozyme dimer present during in vitro fertilization of oocytes on the subsequent embryo development

### Results:

Ad A.: The experimental results indicated that all tested concentrations of lysozyme dimer within the range of 0.1 to 100 µg lysozyme dimer per ml cell or tissue culture suspension, respectively, impaired the cumulus oophorus expansion (mucification), whereas a decrease of oocytes maturation rate was observed only at higher concentrations of the substance, i.e. at concentrations above 10 µg lysozyme dimer per ml. The lysozyme dimer concentration of up to 10 µg/ml had a significant beneficial effect on the oocytes maturation despite of the observed impairing effect on the cumulus mucification. The results of the in vitro fertilization and embryo development of oocytes maturated in the presence of different lysozyme dimer concentrations showed a maximum increase of the fertilization rate and of the number of oocytes that reached the metaphase II stage after 36 hours of incubation, when the lysozyme dimer was used in a concentration of 10 µg/ml. Also the percentage of fertilized oocytes that reached the morula and blastocyst stages was significantly higher when fertilization took place in the presence of 0.1 to 10 µg lysozyme dimer/ml (Fig. 1).

Ad.B.: PMNs were incubated for 3 hours in RPMI - 1640 medium either with the addition of LPS and lysozyme dimer or without these substances. After the incubation the culture medium (supernatant) was collected and frozen at a temperature of -80 °C until used. In another experiment lymphocytes were incubated for 24 hours with the addition of Con A, PHA and lysozyme dimer, then the culture medium was collected and stored as mentioned above. Various experiments were conducted in which the following was evaluated:
B.I. The effect of different concentrations of the supernatant from the PMNs culture on the oocytes maturation. The results indicated that the supernatant from LPS-stimulated PMNs cultured in the presence of lysozyme dimer significantly increased the oocytes maturation rate.
B.II. The influence of a co-culture of 10⁵ LPS-stimulated or not stimulated PMNs in the presence of lysozyme dimer on the maturation of oocytes. The experimental results indicated that the addition of lysozyme dimer to LPS activated PMNs negatively affected the in vitro maturation of the bovine oocytes under the applied experimental conditions. This effect seemed to indicate an increased production of some factor(s) by the PMN cells which affect the oocytes maturation.
B.III. The influence of a supernatant from a PMNs culture - similar to the one disclosed in B.I - on oocytes maturation, fertilization and subsequent embryo development in vitro. It was found that the addition of the supernatant of a cell culture of LPS-stimulated PMNs grown in the presence of lysozyme dimer to the maturation medium at a concentration of 2.5 % v/v had a beneficial effect on oocytes fertilization and embryo development .
B.IV. The effect of a supernatant derived from a culture of lymphocytes grown in the presence of Con A, PHA and lysozyme dimer on the oocytes maturation, fertilization and subsequent embryo development. The results showed that a concentration of 10% v/v of the supernatant in the maturation medium had a beneficial effect on the oocytes fertilization and subsequent embryo development. This effect was lower in the case of lymphocytes stimulated with Con A or PHA with the addition of lysozyme dimer.
Ad.C.: A lysozyme dimer concentration of 10 µg/ml in the medium in which oocytes fertilization took place had a beneficial effect on the quality of embryos obtained in vitro. It remains to be further clarified whether this effect resulted from this substance's influence on the oocytes or on the spermatozoa involved in the fertilization process.

In general, it was found that the lysozyme dimer when used in low concentrations according to the different experimental protocols showed a beneficial effect on in vitro bovine oocytes maturation, their fertilization and subsequent embryo development.

### Example 7: Effect of lysozyme dimer (KLP-602) on the survival of eukaryotic cells

The study was conducted on primary chicken embryo fibroblasts, and on continuous cell lines CC81 (xenotropic cat cell line, TBTR (bovine tracheal cells) and MDBK (bovine kidney cells). Its main objective was to compare the survival time of the cell lines in serum-free Parker's medium with and without the addition of lysozyme dimer.

Primary chicken embryo fibroblasts and continuous cell lines were propagated according to routine methods employed in virology. In brief:
a) Primary chicken embryo fibroblasts: embryos 9-10 days old were cut into small pieces and dissociated into a suspension of single cells by treatment with dilute trypsin (0.25%) in saline solution at pH 7.5 and stirred. The first extraction was discarded, while subsequent extractions were retained. The cells were removed by centrifugation and washed. They were then counted in a hemocytometer chamber and diluted in growth medium (composition see below) to a concentration of about 2.5 x 10⁵ cells/ml. Then the cell suspension was distributed into suitable culture vessels (Roux flasks and/or test tubes) and kept at 37°C. Usually after 24 hours, when monolayer cultures were gained, the growth medium was discarded and monolayers were inoculated with distemper virus, washed and covered by maintaining medium (see composition below) comprising lysozyme dimer.
b) Continuous cell lines: Monolayer cultures on bottles were subcultured by trypsinization (trypsin Difco 0.5%, sodium versene 0.02%) and the cells were suspended in growth medium. As soon as monolayer cultures were gained the growth medium was replaced by maintaining medium. Virus inoculation was carried out before the cell suspension was distributed to glass vessels.
   Growth Medium: Eagle's minimum essential medium supplemented with Hanks' salts and L-glutamine without sodium bicarbonate (Sigma); 4-8% wt. (depending on cell culture used) of fetal calf serum (Sigma; inactivated at 58°C for 30 min); antibiotics (100 units/ml penicillin, 100 µg/ml dihydrostreptomycin sulfate, 2.5 µg/ml amphotericin B; 100x tylosin).
   Maintaining medium: Parker's 199 Medium (Biofactory of Sera and Vaccines, Lublin, Poland); antibiotics (same as in growth medium).
   Toxicity of drugs: the toxicity of lysozyme dimer (KLP 602) and pentoxyfylline was evaluated on cell cultures in bacteriological test tubes (6 tubes per each solution of the drug) and plastic plates ( 8 wells for each solution). The plates with the cultures were kept at 37°C in a humid chamber with carbon dioxide. The effect of the drugs on cells was examined under a light microscope ( a simple or reverse microscope).
   Assessment of cell vitality: Confluent monolayers were detached by trypsinization, centrifuged and the cell suspension was stained with neutral red. The number of cells was counted in a Burker's chamber.
   Viruses:
      1. Attenuated strain BMD of canine parvovirus (Lublin, Poland)
      2. BP1 and BP2 strains of distemper virus (Pulawy, Poland)
   Hemagglutination was performed with erythrocytes of pigs (0.25%) suspended in Sorensen's buffer with 0.1% of bovine albumin, pH 5.8.
   In this study the lysozyme dimer was applied at various concentrations ranging from 1 to 1000 µg/ml (Tables 4, 4a, 4b, 4c).

As a result of the experiments it was found that lysozyme dimer when used at concentrations between 1 and 25 µg per ml cell culture suspension was not only non-toxic to the cells but also remarkably prolonged the survival times of the cells: the cells persisted in the form of unchanged monolayers for 2 to 8 days longer than the untreated controls (Tab.4). When applied at higher concentrations the lysozyme dimer appeared to be slightly toxic to the eukaryotic cells (as concluded from an increased count of dead cells and from morphological changes of the treated cells) but still retained its exciting cell-life extending activity over the whole range (i.e. 1 to 1000 µg per ml cell culture suspension) of tested lysozyme dimer concentrations (Tab.4a, Tab.4b).

This effect of eukaryotic cell life prolongation was also observed with virus-infected animal cells exposed to varying concentrations of lysozyme dimer in combination with a fixed concentration of pentoxyphylline (Tab.4c). Interestingly, in this cell culture experiment an extremely strong effect was observed with chicken embryo fibroblasts at the highest dose of lysozyme dimer applied (i.e. 1000 µg per ml cell culture). Cell life prolongation at a ratio of at least 1.3 (=30 % increase of cell life time compared to controls) and up to 1.67 ( = 67% increase) at the extreme was regularly achieved in the experiments.

**Table 4:**

| Effect of lysozyme dimer (KLP-602) on the survival of eukaryotic cell lines in serum-free Parker's 199 medium | | | | |
|---|---|---|---|---|
| Concentration of lysozyme dimer [µg/ml] | Prolongation of survival time [days] in relation to untreated controls | | | |
| | Chick embryo fibroblasts | Cell line CC81 | Cell line TBTR | Cell line MDBK |
| 1.0 | 6 | 0 | 3 | 2 |
| 2.5 | 6 | 1 | 3 | 2 |
| 5.0 | 6 | 4 | 6 | 2 |
| 10.0 | 6 | 3 | 8 | 0 |
| 25.0 | ND* | 3 | ND | ND |
| 50.0 | ND | T** | ND | ND |
| 100.0 | ND | T | ND | ND |

| | | | | |
|---|---|---|---|---|
| * = not determined; | | | | |
| ** = toxic effects occurred | | | | |

**Table 4a:**

| Effect of lysozyme dimer (KLP 602) on the survival of chick embryo fibroblasts in serum-free Parker's 199 medium | | | | |
|---|---|---|---|---|
| Concentration of lysozyme dimer [µg/ml] | Morphological changes | Dead cells [%] | Survival time [days] | Remarks |
| 8 | BZ | 19.4 | 18 | |
| 16 | BZ | 18.0 | 18 | |
| 32 | BZ | 20.0 | 18 | |
| 64 | BZ | 19.5 | 18 | |
| 125 | BZ | 21.0 | 18 | Slight growth inhibition within first 24 hours |
| 250 | + | 24.0 | 18 | |
| 500 | + + | 21.0 | 18 | growth inhibition within first 24 hours, granular foci |
| 1000 | + + + | 25.0 | 21 | growth inhibition within first 24 h, high percentage of granules |
| Control | BZ | 19.5 | 14 | |
| + minute toxic changes + + foci of toxic changes + + + distinct toxic changes BZ cell morphology without changes | | | | |

**Table 4b:**

| Effect of lysozyme dimer (KLP-602) on the survival of CC81 cells in serum-free Parker's 199 medium | | | |
|---|---|---|---|
| Concentration of lysozyme dimer [µg/ml] | Morphological changes | Dead cells [%] | Survival time [days] |
| 10 | BZ | 11.0 | 10 |
| 20 | BZ | 15.0 | 10 |
| 40 | BZ | 17.0 | 10 |
| 50 | BZ | 17.0 | 10 |
| 80 | + | 24.0 | 10 |
| 100 | + + | 52.0 | 10 |
| Control | BZ | 8.0 | 6 |

**Table 4c:**

| Effect of various doses of lysozyme dimer (KLP-602) in combination with 10 µg/ml pentoxyfylline on the survival of virus infected cultures of chicken embryo fibroblasts and CC81 cells in serum-free Parker's 199 medium | | |
|---|---|---|
| 10 µg/ml pentoxyfylline + lysozyme dimer [µg/ml] | Survival of distemper virus / canine parvovirus infected cells [days] | |
| | Chick embryo fibroblasts | Cell line CC81 |
| 8 | 21/18 | NB |
| 10 | NB | 13/10 |
| 16 | NB | NB |
| 20 | NB | 13/10 |
| 32 | 21/18 | NB |
| 40 | NB | 13/10 |
| 50 | NB | 13/10 |
| 64 | 21/18 | NB |
| 80 | NB | 13/10 |
| 100 | NB | 13/10 |
| 125 | 21/18 | NB |
| 250 | 21/18 | T |
| 500 | 25/18 | T |
| 1000 | 30/21 | T |
| Control | 18/14 | 13/10 |
| T = toxic action of the drugs; NB = not determined | | |

Infection was done with distemper virus and canine parvovirus; viral titers of controls (in CCID₅₀/ml) were 10^{3.25} (distemper virus) / 10^{5.0} (canine parvovirus)

Investigations carried out with other cells under comparable conditions, in particular with Leydig cells and similar macrophages, confirmed the aforementioned results. They further revealed that the cells' DNA content and protein synthesis increased after treatment with lysozyme dimer. It is, however, not yet fully understood how the lysozyme dimer interacts with eukaryotic cells and particularly with mammalian immunocompetent cells in order to interfere with apoptosis, i.e. the genetically pre-determined "programmed" cell death. The experimental results obtained so far are nevertheless very exciting with regard to the observed anti-aging effect of the lysozyme dimer on eukaryotic cells and the practical implications thereof may not yet be entirely anticipated.

## Claims

1. Use of a lysozyme dimer for the manufacture of a pharmaceutical composition for the prophylactic intervention or therapeutic treatment of animal or human fertility impairment.

2. Use according to claim 1, wherein said prophylactic intervention or therapeutic treatment comprises improving at least one of the following fertility parameters: sperm quality, egg cell maturation, embryo development.

3. Use according to claim 1 or 2, wherein said prophylactic intervention or therapeutic treatment comprises improving at least one of the following sperm quality factors: duration of sperm cell viability, sperm cell motility, sperm cell membrane stability, acrosome protection, concentration of morphologically normal sperm cells, total count of sperm cells in the ejaculate.

4. Use according to claim 1, wherein said impairment is affected or caused by at least one of the following disorders: immunological disorder, hormonal disorder, metabolic disorder, and wherein the immunological disorder optionally comprises an inflammation of the uterus.

5. Use according to claim 1, wherein said fertility impairment is due to oligospermia.

6. Use according to claim 1, wherein said prophylactic intervention or therapeutic treatment comprises modulating at least one of the following parameters: cellular immunity, phagocytic activity, a cytokine level, a hormone level.

7. Use according to anyone of claims 1 to 6, wherein said pharmaceutical composition comprises lysozyme dimer in an amount suitable for administering said lysozyme dimer to a human or animal individual in a single or repeated dose of about 0.005 to 5, preferably about 0.01 to 1, mg lysozyme dimer per kg body weight, or in a single or repeated dose of about 0.07 to 70, preferably 0.14 to 14, µg lysozyme dimer perml blood.

8. Use according to anyone of claims 1 to 7, wherein said composition is in the form of a liquid solution containing the lysozyme dimer at a concentration of 0.01 to 10, preferably 0.1 to 0.5, mg/ml of the solution.

9. Use according to claim 1 or 2, for improving egg cell maturation and/or embryo development wherein said prophylactic intervention or therapeutic treatment comprises supplementing an egg cell maturation medium with an effective amount of lysozyme dimer, preferably with about 1 to 10 µg lysozyme dimer per ml of maturation medium.

10. Use of a lysozyme dimer for an improved preservation of eukaryotic cells, particularly of animal or human germ cells, and/or for prolongation of eukaryotic cell life, by incubating or storing eukaryotic cells in the presence of lysozyme dimer, preferably in the presence of 0.1 to 50 µg lysozyme dimer per ml of a suspension containing said cells.

11. Use according to claim 10, for a prolongation of cell life by a factor of at least up to 1.3, preferably by a factor of up to 1.67.

12. Use according to anyone of the preceding claims, wherein said lysozyme dimer is a crosslinked dimerization product of lysozyme monomers and contains about 10 % by weight or less of undesired byproducts, particularly of monomeric and oligomeric forms of lysozyme.

13. A method for an improved preservation of eukaryotic cells, particularly of animal or human germ cells, and/or for prolongation of eukaryotic cell life, wherein the method comprises storing or incubating said cells in the presence of lysozyme dimer, preferably in the presence of 0.1 to 50 µg lysozyme dimer per ml of a suspension containing said cells.

14. The method according to claim 13, wherein said preservation comprises a freezing step and wherein lysozyme dimer is added to the eukaryotic cells, preferably before subjecting the cells to the freezing step, at a dose of 0.1 to 20 µg, preferably of about 1 to 4 µg, per 1 ml of a suspension containing said cells.

15. A method according to claim 13 or 14, wherein said lysozyme dimer is a crosslinked dimerization product of lysozyme monomers and contains about 10 % by weight or less of undesired byproducts, particularly of monomeric and oligomeric forms of lysozyme.

## Patentansprüche

1. Verwendung eines Lysozymdimeren zur Herstellung einer pharmazeutischen Zusammensetzung für den prophylaktischen Eingriff bei oder die therapeutische Behandlung von tierischer oder menschlicher Fruchtbarkeitsbeeinträchtigung.

2. Verwendung nach Anspruch 1, worin der benannte prophylaktische Eingriff oder die benannte therapeutische Behandlung umfasst, zumindest einen der folgenden Fruchtbarkeitsparameter zu verbessern: Samenqualität, Eizellenreifung, Embryoentwicklung.

3. Verwendung nach Anspruch 1 oder 2, worin der benannte prophylaktische Eingriff oder die benannte therapeutische Behandlung umfasst, zumindest einen der folgenden Samenqualitätsfaktoren zu verbessern: Dauer der Samenzellen-Lebensfähigkeit, Samenzellenmotilität, Samenzellen-Membranstabilität, Akrosomenschutz, Konzentration der morphologisch normalen Samenzellen, Gesamtzahl der Samenzellen im Ejakulat.

4. Verwendung nach Anspruch 1, worin die benannte Beeinträchtigung durch zumindest eine der folgenden Störungen beeinflusst oder bewirkt wird: immunologische Störung, hormonale Störung, Stoffwechselstörung, und worin die immunologische Störung wahlweise eine Entzündung der Gebärmutter umfasst.

5. Verwendung nach Anspruch 1, worin die benannte Fruchtbarkeitsbeeinträchtigung auf Oligospermie zurückzuführen ist.

6. Verwendung nach Anspruch 1, worin der benannte prophylaktische Eingriff oder die benannte therapeutische Behandlung umfasst, zumindest einen der folgenden Parameter zu modulieren: zellgebundene Immunität, Phagozytenaktivität, einen Cytokinspiegel, einen Hormonspiegel.

7. Verwendung nach einem beliebigen der Ansprüche 1 bis 6, worin die benannte pharmazeutische Zusammensetzung Lysozymdimer in einer Menge umfasst, die zur Verabreichung des benannten Lysozymdimers an einen einzelnen Menschen oder an ein einzelnes Tier in einer einzigen oder wiederholten Dosis von etwa 0,005 bis 5, bevorzugt etwa 0,01 bis 1 mg Lysozymdimer pro kg Körpergewicht oder in einer einzigen oder wiederholten Dosis von etwa 0,07 bis 70, bevorzugt 0,14 bis 14 µg Lysozymdimer pro ml Blut geeignet ist.

8. Verwendung nach einem beliebigen der Ansprüche 1 bis 7, worin die benannte Zusammensetzung in Gestalt einer flüssigen Lösung vorliegt, die das Lysozymdimer in einer Konzentration von 0,01 bis 10, bevorzugt 0,1 bis 0,5 mg/ml der Lösung enthält.

9. Verwendung nach Anspruch 1 oder 2 zur Verbesserung der Eizellenreifung und/oder Embryoentwicklung, worin der benannte prophylaktische Eingriff oder die benannte therapeutische Behandlung umfasst, ein Eizellenreifungsmedium mit einer wirksamen Menge von Lysozymdimer zu ergänzen, bevorzugt mit etwa 1 bis 10 µg Lysozymdimer pro ml des Reifungsmediums.

10. Verwendung eines Lysozymdimers für eine verbesserte Erhaltung von eukaryotischen Zellen, insbesondere von tierischen oder menschlichen Keimzellen, und/ oder für eine Verlängerung der Lebensdauer der eukaryotischen Zellen durch die Inkubation oder Aufbewahrung von eukaryotischen Zellen in Gegenwart von Lysozymdimer, bevorzugt in Gegenwart von 0,1 bis 50 µg Lysozymdimer pro ml einer die benannten Zellen enthaltenden Suspension.

11. Verwendung nach Anspruch 10 für eine Verlängerung der Zellenlebensdauer um einen Faktor von mindestens bis zu 1,3, bevorzugt um einen Faktor von bis zu 1,67.

12. Verwendung nach einem beliebigen der vorangehenden Ansprüche, worin das benannte Lysozymdimer ein vernetztes Dimerisationsprodukt von Lysozymmonomeren ist und etwa 10 Gewichtsprozent oder weniger an unerwünschten Nebenprodukten, insbesondere monomeren und oligomeren Formen von Lysozym, enthält.

13. Verfahren zur verbesserten Erhaltung von eukaryotischen Zellen, insbesondere von tierischen oder menschlichen Keimzellen, und/oder für eine Verlängerung der Lebensdauer eukaryotischer Zellen, worin das Verfahren umfasst, die benannten Zellen in Gegenwart von Lysozymdimer aufzubewahren oder zu inkubieren, bevorzugt in Gegenwart von 0,1 bis 50 µg Lysozymdimer pro ml einer die benannten Zellen enthaltenden Suspension.

14. Verfahren nach Anspruch 13, worin die benannte Erhaltung einen Gefrierschritt umfasst und worin Lysozymdimer zu den eukaryotischen Zellen in einer Dosis von 0,1 bis 20 µg, bevorzugt von etwa 1 bis 4 µg pro 1 ml einer die benannten Zellen enthaltenden Suspension hinzugefügt wird, und zwar bevorzugt, ehe die Zellen dem Gefrierschritt unterworfen werden,.

15. Verfahren nach Anspruch 13 oder 14, worin das benannte Lysozymdimer ein vernetztes Dimerisationsprodukt von Lysozymmonomeren ist und etwa 10 Gewichtsprozent oder weniger an unerwünschten Nebenprodukten, insbesondere monomeren und oligomeren Formen von Lysozym, enthält.

## Revendications

1. Utilisation d'un dimère de lysozyme pour la fabrication d'une composition pharmaceutique destinée à l'intervention prophylactique ou au traitement thérapeutique d'une altération de fertilité animale ou humaine.

2. Utilisation selon la revendication 1, dans laquelle ladite intervention prophylactique ou ledit traitement thérapeutique comprend l'amélioration d'au moins l'un des paramètres de fertilité suivant : qualité du sperme, maturation des ovules, développement de l'embryon.

3. Utilisation selon la revendication 1 ou 2, dans laquelle ladite intervention prophylactique ou ledit traitement thérapeutique comprend l'amélioration d'au moins l'un des facteurs de qualité de sperme suivant : durée de viabilité des spermatozoïdes, mobilité des spermatozoïdes, stabilité de la membrane de spermatozoïde, protection de l'acrosome, concentration en spermatozoïdes morphologiquement normaux, nombre total de spermatozoïdes dans l'éjaculat.

4. Utilisation selon la revendication 1, dans laquelle ladite altération est réalisée ou provoquée par au moins l'un des troubles suivants : trouble immunologique, trouble hormonal, trouble métabolique, et dans lequel le trouble immunologique comprend facultativement une inflammation de l'utérus.

5. Utilisation selon la revendication 1, dans laquelle ladite altération de la fertilité est due à une oligospermie.

6. Utilisation selon la revendication 1, dans laquelle ladite intervention prophylactique ou ledit traitement thérapeutique comprend la modulation d'au moins l'un des paramètres suivants : immunité cellulaire, activité phagocytaire, un niveau de cytokine, un niveau hormonal.

7. Utilisation selon l'une quelconque des revendications 1 à 6, dans laquelle ladite composition pharmaceutique comprend un dimère de lysozyme dans une proportion convenant à une administration dudit dimère de lysozyme à un sujet humain ou animal sous forme d'une dose unique ou d'une dose répétée d'environ 0,005 à 5, de préférence environ 0,01 à 1 mg de dimère de lysozyme par kilogramme de masse corporelle, ou bien sous forme d'une dose unique ou d'une dose répétée d'environ 0,07 à 70, de préférence 0,14 à 14 µg de dimère de lysozyme par millilitre de sang.

8. Utilisation selon l'une quelconque des revendications 1 à 7, dans laquelle ladite composition est sous forme d'une solution liquide contenant le dimère de lysozyme à une concentration de 0,01 à 10, de préférence 0,1 à 0,5 mg/ml de la solution.

9. Utilisation selon la revendication 1 ou 2, destinée à améliorer la maturation des ovules et/ou le développement de l'embryon, dans laquelle ladite intervention prophylactique ou ledit traitement thérapeutique comprend l'addition d'un milieu de maturation des ovules comprenant une quantité efficace de dimère de lysozyme, comprenant de préférence environ 1 à 10 µg de dimère de lysozyme par millilitre de milieu de maturation.

10. Utilisation d'un dimère de lysozyme en vue d'une conservation améliorée de cellules eucaryotiques, en particulier de cellules germinales animales ou humaines, et/ou en vue de la prolongation de la durée de vie des cellules eucaryotiques, en incubant ou en stockant des cellules eucaryotiques en présence de dimère de lysozyme, de préférence de 0,1 à 50 µg de dimère de lysozyme par ml d'une suspension contenant lesdites cellules.

11. Utilisation selon la revendication 10, en vue d'une prolongation de la durée de vie des cellules d'un facteur allant jusqu'à au moins 1,3, de préférence d'un facteur allant jusqu'à 1,67.

12. Utilisation selon l'une quelconque des revendications précédentes, dans laquelle ledit dimère de lysozyme est un produit de dimérisation réticulée de monomères de lysozyme et qui contient environ 10 % en poids ou moins de sous-produits indésirés, en particulier de formes monomère et oligomère de lysozyme.

13. Procédé destiné à une conservation améliorée de cellules eucaryotiques, en particulier de cellules germinales animales ou humaines, et/ou destiné à la prolongation de la durée de vie des cellules eucaryotiques, dans lequel le procédé comprend le stockage ou l'incubation desdites cellules en présence de dimère de lysozyme, de préférence en présence de 0,1 à 50 µg de dimère de lysozyme par millilitre d'une suspension contenant lesdites cellules.

14. Procédé selon la revendication 13, dans lequel ladite conservation comprend une étape de congélation et dans lequel du dimère de lysozyme est ajouté aux cellules eucaryotiques, de préférence avant de soumettre les cellules à l'étape de congélation, à une dose de 0,1 à 20 µg, de préférence de 1 à 4 µg pour 1 ml d'une suspension contenant lesdites cellules.

15. Procédé selon la revendication 13 ou 14, dans lequel ledit dimère de lysozyme est un produit de dimérisation réticulée de monomère de lysozyme et qui contient environ 10 % en poids ou moins de sous-produits indésirés, en particulier de formes monomère et oligomère de lysozyme.
